# EUROPEAN PATENT SPECIFICATION

(11) **EP 4 054 671 B1**
(45) Date of publication and mention of the grant of the patent: **11.03.2026**
(21) Application number: 20801114.8
(22) Date of filing: 15.10.2020
(51) Int. Cl.: A61M 1/36

(54) **TECHNIQUES FOR IMAGE-BASED EXAMINATION OF DIALYSIS ACCESS SITES**
VERFAHREN ZUR BILDBASIERTEN UNTERSUCHUNG VON DIALYSEZUGANGSSTELLEN
TECHNIQUES D'EXAMEN FONDÉ SUR L'IMAGE DE SITES D'ACCÈS POUR DIALYSE

(30) Priority: 08.11.2019 US 201916678234
(43) Date of publication of application: 14.09.2022
(73) Proprietor: Fresenius Medical Care Holdings, Inc., Waltham, MA 02451 (US)
(72) Inventor: ZHANG, Hanjie, Waltham, Indiana 02451 (US); KOTANKO, Peter, Waltham, Massachusetts 02451 (US); CHARETTE, Leslie A., Waltham, Massachusetts 02451 (US); MUCHIUTTI, Carlos, Waltham, Massachusetts 02451 (US); SOR, Murat, Waltham, Massachusetts 02451 (US); KOH, Elsie, Waltham, Massachusetts 02451 (US); MADDUX, Dugan W., Waltham, Massachusetts 02451 (US); USVYAT, Len, Waltham, Massachusetts 02451 (US)
(74) Representative: Freischem & Partner Patentanwälte mbB
(86) International application number: PCT/US2020/055674
(87) International publication number: WO 2021/091668

(56) References cited:
- DE-A1- 102017 130 107
- US-A1- 2016 125 161
- US-A1- 2017 119 258
- US-B2- 7 995 816

## Description

### FIELD

The disclosure generally relates to processes for examining physical characteristics of a portion of patient based on images of the portion, and, more particularly, to techniques for assessing a condition of a dialysis access site of a patient.

### BACKGROUND

Dialysis treatment requires access to the patient circulatory system via a dialysis access site in order to process patient blood using a dialysis treatment unit. For peritoneal dialysis (PD), the dialysis access site may be via a catheter. Hemodialysis (HD) treatment requires access to blood circulation in an extracorporeal circuit connected to the main cardiovascular circuit of the patient through a vascular or arteriovenous (AV) access. Typical HD access types may include arteriovenous fistula (AVF) and arteriovenous graft (AVG). During an HD treatment, blood is removed from the vascular access by an arterial needle fluidly connected to the extracorporeal circuit and provided to an HD treatment unit. After processing via the HD treatment unit, the blood is sent back to the vascular access through a venous needle and back into the patient cardiovascular circuit.

Accordingly, the health of the access site of a patient is of primary importance to the efficacy of the dialysis treatment. For example, a vascular access should be capable of providing adequate blood flow for HD treatment and should be free of serious complications, such as severe pain and/or swelling, aneurysms, and/or the like. Conventional vascular access site monitoring techniques typically require visual inspection of the site by a healthcare professional capable of providing a diagnosis and treatment recommendation. Such monitoring requires either a patient visit to a healthcare facility and/or a home visit by a healthcare professional. In addition, although knowledgeable, the healthcare professional generally does not have access to a robust library of patient treatment outcomes for determining an optimized treatment recommendation. Accordingly, conventional monitoring techniques are inefficient and burdensome to the patient, particularly for patients receiving treatments at home.

It is with respect to these and other considerations that the present improvements may be useful.

It is referred to US 2017/0119258 A1 and US 7 995 816 B2 as prior art.

### SUMMARY

This Summary is provided to introduce a selection of concepts in a simplified form that are further described below in the Detailed Description. This Summary is not intended to necessarily identify key features or essential features of the claimed subject matter, nor is it intended as an aid in determining the scope of the claimed subject matter. The invention is defined by the appended claims.

In accordance with various aspects of the described embodiments is an apparatus that may include at least one processor and a memory coupled to the at least one processor. The memory may include instructions that, when executed by the at least one processor, cause the at least one processor to receive an access site image comprising at least one image of a dialysis access site of a patient, determine access site information for the dialysis access site based on at least one access site feature determined from the access site image, the access site information indicating a condition of the dialysis access site, and determine a treatment recommendation for the dialysis access site based on the access site information.

In some embodiments of the apparatus, the instructions, when executed by the at least one processor, may cause the at least one processor to receive access site description information, and determine the access site information based on the at least one access site feature and the access site description information. In various embodiments of the apparatus, the dialysis access site comprising one of an arteriovenous fistula (AVF) or an arteriovenous graft (AVG).

In some embodiments of the apparatus, the instructions, when executed by the at least one processor, may cause the at least one processor to provide the access site image to a computational model to determine the at least one access site feature. In exemplary embodiments of the apparatus, the instructions, when executed by the at least one processor, may cause the at least one processor to provide the access site information to a computational model to determine the treatment recommendation.

In some embodiments of the apparatus, the at least one access site feature may include at least one of size, color, shape, or presence of an abnormality. In various embodiments of the apparatus, the access site image captured via a client computing device. The instructions, when executed by the at least one processor, cause the at least one processor to determine a classification of the access site based on access site classification information. In various embodiments of the apparatus, the classification may include a score and at least one treatment action. In exemplary embodiments of the apparatus, the treatment recommendation may include analytics information indicating at least one treatment outcome associated with the treatment recommendation.

In accordance with various aspects of the described embodiments is a method, that may include receiving an access site image comprising at least one image of a dialysis access site of a patient, determining access site information for the dialysis access site based on at least one access site feature determined from the access site image, the access site information indicating a condition of the dialysis access site, and determining a treatment recommendation for the dialysis access site based on the access site information.

In some embodiments of the method, the method may include receiving access site description information, and determining the access site information based on the at least one access site feature and the access site description information. In some embodiments of the method, the dialysis access site may include one of an arteriovenous fistula (AVF) or an arteriovenous graft (AVG).

In some embodiments of the method, the method may include providing the access site image to a computational model to determine the at least one access site feature. In some embodiments of the method, the method may include providing the access site information to a computational model to determine the treatment recommendation.

In some embodiments of the method, the at least one access site feature may include at least one of size, color, shape, or presence of an abnormality. In some embodiments of the method, the access site image may be captured via a client computing device. The method includes determining a classification of the access site based on access site classification information. In some embodiments of the method, the classification may include a score and at least one treatment action. In some embodiments of the method, the treatment recommendation may include analytics information indicating at least one treatment outcome associated with the treatment recommendation.

### BRIEF DESCRIPTION OF THE DRAWINGS

By way of example, specific embodiments of the disclosed machine will now be described, with reference to the accompanying drawings, in which:
**FIG. 1** illustrates a first exemplary operating environment in accordance with the present disclosure;
**FIG. 2** illustrates exemplary access site classification information in accordance with the present disclosure;
**FIG. 3** illustrates a second exemplary operating environment in accordance with the present disclosure;
**FIG. 4** illustrates a third exemplary operating environment in accordance with the present disclosure;
**FIG. 5** illustrates a logic flow in accordance with the present disclosure; and
**FIG. 6** illustrates an embodiment of a computing architecture in accordance with the present disclosure.

### DETAILED DESCRIPTION

The present embodiments will now be described more fully hereinafter with reference to the accompanying drawings, in which several exemplary embodiments are shown. The subject matter of the present disclosure, however, may be embodied in many different forms and should not be construed as limited to the embodiments set forth herein. Rather, these embodiments are provided so that this disclosure will be thorough and complete, and willfully convey the scope of the subject matter to those skilled in the art. In the drawings, like numbers refer to like elements throughout.

As described above, dialysis treatment requires at least one dialysis access site for accessing the circulatory system of a patient. Peritoneal dialysis (PD) may use an access site that includes a PD catheter. Hemodialysis (HD) may use an access site that includes an arteriovenous (AV) fistula (AVF), AV graft (AVG), or an HD catheter. An AVF is an artery surgically connected to a vein, while an AVG is a surgically placed conduit of synthetic material connecting an artery to a vein.

The health of the access site is paramount to a successful dialysis treatment. Monitoring of an access site may involve determining various characteristics of the access site that may indicate complications, abnormalities, and/or the like. Non-limiting examples of access site characteristics may include blood flow rate, color, size, shape, presence and/or severity of pain, inflammation, aneurysm, venous stenosis, thrombosis, and/or the like. In addition, monitoring may include determining variances between current access site characteristics and previous access site characteristics, determining access site trends (for instance, whether inflammation is increasing or decreasing), and/or the like. Based on access site characteristics, a treatment plan may be determined to monitor and/or treat access site abnormalities.

Conventional methods for monitoring and evaluating patient access site characteristics typically involve clinical monitoring via physical examination of the access site by a healthcare professional. Clinical evaluation may include visual inspection, palpation, and/or auscultation. Such clinical monitoring requires that the patient visit a healthcare facility and/or a healthcare professional visit the patient at their home. Requiring physical evaluation is burdensome to the patient and is difficult to perform, particularly over short periods (for instance, daily) as may be required by a serious condition. In addition, patient compliance with follow-up instructions for monitoring an abnormal access site may be relatively low when they are required to visit a healthcare facility and/or receive a visit from a healthcare professional.

If an abnormality is detected, the healthcare professional may recommend a treatment and/or further evaluation by an experienced physician. Although physicians and other healthcare professionals are experienced in diagnosing and treating access site abnormalities, they do not have access to a robust repository of population-based treatment outcomes that may allow them to more effectively arrive at a treatment option.

Accordingly, some embodiments may provide processes for image-based examination of dialysis access sites using population-based treatment information. For example, in various embodiments, an access site analysis process may receive an image of an access site. For instance, a patient may take an image of their access site using a personal computing device (for example, a smartphone, a tablet computing device, and/or the like) and send the image to an access site analysis platform. The access site analysis process may process the image using a computational model to determine access site features, such as size, color, presence of abnormalities, and/or the like.

In some embodiments, the patient may provide access site description information that may be associated with the image. In general, access site description information may include information describing or otherwise indicating characteristics of the access site, such as the presence and/or severity of pain, inflammation, aneurysm, and/or the like. The access site analysis process may provide the access site features and/or access site description information to a computational model to determine a treatment recommendation for the access site based on the access site features and/or access site description information. In various embodiments, computational models used by the access site analysis process may be trained using actual patient information and/or images of an individual patient and/or a patient population (for instance, of chronic kidney disease (CKD) and/or end-stage renal disease (ESRD) patients).

In some embodiments, the access site analysis process may be used to remotely monitor, analyze, trend, and/or the like a patient's access site by using a combination of digital imaging, trending, intervention and outcome information to improve the longevity and care of a patient's access site. In some embodiments, the access site analysis process may be an internet-based, Software-as-a-Service (SaaS), and/or cloud-based platform that may be used by a patient or a healthcare team to monitor patients clinical care and can be used to provide expert third-party assessments, for example, as a subscription or other type of service to healthcare providers.

For example, the access site analysis process may operate in combination with a "patient portal" or other type of platform that a patient and healthcare team may use to exchange information. For instance, dialysis treatment centers mange in-home patients who receive treatment in their own home and in-center patients who receive treatment at a treatment center. The patients may be in various stages of renal disease, such as chronic kidney disease (CKD), end-stage renal disease (ESRD), and/or the like. In-home patients may take a picture of their access site, such as an catheter site, AVF site, AVG site, and/or the like, using a smartphone or other personal computing device on a periodic basis (for instance, daily, weekly, monthly, and/or the like) or as necessary (for instance, based on the appearance and/or change of an abnormality). The image may be uploaded to a patient portal or other platform and routed to a dialysis access site analysis system operative to perform the access site analysis process according to some embodiments. Similarly, pictures of the access sites of in-center patients may be taken by the patient and/or clinical staff and uploaded to the patient portal for access by the access site analysis system.

In some embodiments, patient images may be stored in a repository or other database, including, without limitation, a healthcare information system (HIS), electronic medical record (EMR) system, and/or the like. Images in the repository may be catalogued and indexed by patient including key clinical information, demographics, medical history, and/or the like to be processed by the access site analysis system at a patient level and/or a population level. Use of patient image information at a population level may require de-identification of protected health information (PHI) and/or other information capable of identifying a patient according to required regulations, protocols, and/or the like, such as Health Insurance Portability and Accountability Act of 1996 (HIPAA).

The access site analysis system may operate to compare a patient's most recent image to the patient's previous images to automatically spot trends and variances in the patient's access site using imaging analysis technology configured according to some embodiments. Variances and/or trends may involve various access site characteristics including, without limitation, color, size, shape, placement of the patient's access, skin characteristics, vascular system characteristics, patient-reported information such as touch sensitivity, pulse, temperature, pain, and/or the like. In some embodiments, the access site analysis system may provide an assessment or diagnosis and/or one or more treatment recommendations, which may be provided to a healthcare team.

The healthcare team may then review the recommendations and either accept, decline, or revise the intervention for the patient. Healthcare team interventions may be documented and stored in the repository on both a patient-level and a population-level so that they can be followed to monitor success rates and outcomes to provide further training data to computational models used according to some embodiments.

Accordingly, the access site analysis system may use computational models that may continuously learn and monitor outcomes and success rates and provide feedback, treatment recommendations, diagnoses, and/or the like to the clinical care team using population-level analytics. The population-level analytics may be segmented based on various properties, such as age, gender, disease state, national population, regional population, access site type, access site condition or abnormalities, and/or the like.

For example, the access site analysis system may be capable of providing a recommended treatment based on information associated with patients with a similar medical history and access site abnormality, including, for instance: Intervention Recommendation 1, which was attempted on N number of patients and had a 40% success rates on similar patients; Intervention Recommendation 2, which had a 25% success rates on similar patients; and/or Intervention Recommendation 3, which was attempted on X number of patients in your geographic region and had a 80% success rates on similar patients.

In addition, some embodiments may provide processes for automated classification of access site conditions. For example, various embodiments may include an access site analysis process operative to classify the stages of access site aneurysms, such as AVF aneurysms. As described previously, conventional systems typically require in-person visual inspection of the aneurysm or other abnormality. In various embodiments, patient- or healthcare provider-captured images of the access site may be analyzed via a computational model operative to determine a classification, stage, categorization, or other definition for the access site. For example, access sites may be categorized on a scale of 0 (little to no health risk) to 3 (urgent care required). In this manner, the access site analysis process may be operable to automatically classify patient access sites, such as AVFs and/or AVGs, and suggest actions when necessary, thereby reducing or even eliminating the burden on human healthcare professionals to perform these tasks and provide timely diagnosis during an in-person patient visit.

Therefore, dialysis access site analysis processes according to some embodiments may provide multiple technological advantages and technical features over conventional systems, including improvements to computing technology. One non-limiting example of a technological advantage may include examining access sites using automated processes of digital images employing, for example, artificial intelligence (AI) and/or machine learning (ML) processes. Another non-limiting example of a technological advantage may include allowing remote analysis of a patient access site without requiring an in-person visual inspection by a healthcare professional, reducing or even eliminating the need for a visit to/from the healthcare professional by/to the patient. In a further non-limiting example of a technological advantage, access site analysis processes according to some embodiments may determine a course of treatment for an access site condition using population-based patient outcome and success rates for the same or similar conditions as determined by an AI and/or ML computational model. Other technological advantages are provided in this Detailed Description. Embodiments are not limited in this context.

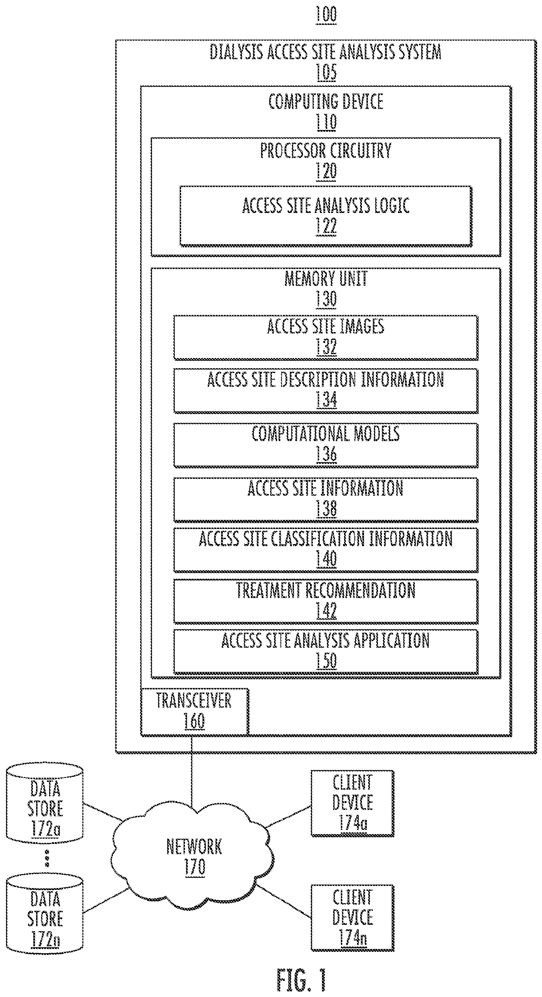

**FIG. 1** illustrates an example of an operating environment 100 that may be representative of some embodiments. As shown in FIG. 1, operating environment 100 may include a dialysis access site analysis system 105. In various embodiments, dialysis access site analysis system 105 may include a computing device 110 communicatively coupled to network 170 via a transceiver 160. In some embodiments, computing device 110 may be a server computer or other type of computing device.

Computing device 110 may be configured to manage, among other things, operational aspects of an access site analysis process according to some embodiments. Although only one computing device 110 is depicted in FIG. 1, embodiments are not so limited. In various embodiments, the functions, operations, configurations, data storage functions, applications, logic, and/or the like described with respect to computing device 110 may be performed by and/or stored in one or more other computing devices (not shown), for example, coupled to computing device 110 via network 170 (for instance, one or more of client devices 174a-n). A single computing device 110 is depicted for illustrative purposes only to simplify the figure. Embodiments are not limited in this context.

Computing device 110 may include a processor circuitry that may include and/or may access various logics for performing processes according to some embodiments. For instance, processor circuitry 120 may include and/or may access an access site analysis logic 122. Processing circuitry 120, access site analysis logic 122, and/or portions thereof may be implemented in hardware, software, or a combination thereof. As used in this application, the terms "logic," "component," "layer," "system," "circuitry," "decoder," "encoder," "control loop," and/or "module" are intended to refer to a computer-related entity, either hardware, a combination of hardware and software, software, or software in execution, examples of which are provided by the exemplary computing architecture 600. For example, a logic, circuitry, or a module may be and/or may include, but are not limited to, a process running on a processor, a processor, a hard disk drive, multiple storage drives (of optical and/or magnetic storage medium), an object, an executable, a thread of execution, a program, a computer, hardware circuitry, integrated circuits, application specific integrated circuits (ASIC), programmable logic devices (PLD), digital signal processors (DSP), field programmable gate array (FPGA), a system-on-a-chip (SoC), memory units, logic gates, registers, semiconductor device, chips, microchips, chip sets, software components, programs, applications, firmware, software modules, computer code, a control loop, a computational model or application, an AI model or application, an ML model or application, a proportional-integral-derivative (PID) controller, variations thereof, combinations of any of the foregoing, and/or the like.

Although access site analysis logic 122 is depicted in FIG. 1 as being within processor circuitry 120, embodiments are not so limited. For example, access site analysis logic 122 and/or any component thereof may be located within an accelerator, a processor core, an interface, an individual processor die, implemented entirely as a software application (for instance, an access site analysis application 150) and/or the like.

Memory unit 130 may include various types of computer-readable storage media and/or systems in the form of one or more higher speed memory units, such as read-only memory (ROM), random-access memory (RAM), dynamic RAM (DRAM), Double-Data-Rate DRAM (DDRAM), synchronous DRAM (SDRAM), static RAM (SRAM), programmable ROM (PROM), erasable programmable ROM (EPROM), electrically erasable programmable ROM (EEPROM), flash memory, polymer memory such as ferroelectric polymer memory, ovonic memory, phase change or ferroelectric memory, silicon-oxide-nitride-oxide-silicon (SONOS) memory, magnetic or optical cards, an array of devices such as Redundant Array of Independent Disks (RAID) drives, solid state memory devices (e.g., USB memory, solid state drives (SSD) and any other type of storage media suitable for storing information. In addition, memory unit 130 may include various types of computer-readable storage media in the form of one or more lower speed memory units, including an internal (or external) hard disk drive (HDD), a magnetic floppy disk drive (FDD), and an optical disk drive to read from or write to a removable optical disk (e.g., a CD-ROM or DVD), a solid state drive (SSD), and/or the like.

Memory unit 130 may store various types of information and/or applications for an access site analysis process according to some embodiments. For example, memory unit 130 may store access site images 132, access site description information 134, computational models 136, access site information 138, access site classification information 140, treatment recommendations 142, and/or an access site analysis application 150. In some embodiments, some or all of access site images 132, access site description information 134, computational models 136, access site information 138, access site classification information 140, treatment recommendations 142, and/or an access site analysis application 150 may be stored in one or more data stores 172a-n accessible to computing device 110 via network 170. For example, one or more of data stores 172a-n may be or may include a HIS, an EMR system, a dialysis information system (DIS), a picture archiving and communication system (PACS), a Centers for Medicare and Medicaid Services (CMS) database, U.S. Renal Data System (USRDS), a proprietary database, and/or the like.

In some embodiments, access site analysis logic 122, for example, via access site analysis application 150, may operate to analyze patient access site images 132 to determine access site information 138 (for instance, a diagnosis) and/or treatment recommendation 142 using one or more computational models 136. Access site images 132 may include a digital or other electronic file that includes a picture and/or video of an access site and/or other portions of a patient. The images may be stored as image files such as *.jpg, *.png, *.bmp, *.tif, and/or the like. In some embodiments, the images may be or may include video files such as *.mp3, *.mp4, *.avi, and/or the like. A patient, healthcare provider, caretaker, or other individual may capture the image using any capable device, such as a smartphone, tablet computing device, laptop computing device, personal computer (PC), camera, video camera, and/or the like.

A user, such as the patient and/or healthcare professional, may send, transmit, upload, or otherwise provide access site images 132 to access site analysis system 105 via a client device 174a communicatively coupled to computing device 110 via network 170. For example, access site analysis application may be or may include a website, internet interface, portal, or other network-based application that may facilitate uploading digital access site images 132 for storage in memory unit 130 and/or data stores 172a-n. In some embodiments, a patient client device 174a-n may operate a client application (for instance, a mobile application or "app") operative to communicate with access site analysis application 150 for providing access site images 132. In some embodiments, a patient may upload digital access site images 132 via a patient portal of a dialysis clinic or other healthcare provider. Access site analysis application 150 may be communicatively coupled to the patient portal to receive images therefrom. Embodiments are not limited in this context.

In addition, a patient or healthcare provider may provide access site description information 134 describing characteristics of the access site. In general, access site description information 134 may include any type of textual, audio, visual, and/or the like data outside of an access site image 132 that may indicate characteristics of the access site. For example, access site description information 134 may include descriptions regarding pain, swelling, color, size, blood flow information, duration of a condition or characteristic, age of access site, type of access site, patient vitals, and/or the like. In various embodiments, access site description information 134 may be associated with one or more access site images 132, for example, as metadata, related within one or more medical record entries, and/or the like. For instance, access site analysis application 150 may create a record for an access site image 132 that includes or refers to associated access site description information 134. In this manner, access site analysis application 150 may access information describing and/or providing context to an access site image 132.

Access site analysis application 150 may analyze access site images 132 and/or access site description information 134 to determine access site information 138. In general, access site information 138 may include a diagnosis, classification, categorization, access site features, or other analysis result determined from analyzing an access site image 132 and/or access site description information 134. For example, access site information 138 may include access site features of an access site image 132, including, without limitation, color, size, shape, access site elements (for instance, scabbing, bleeding, and/or the like), and/or other information that may be discerned from analyzing an access site image 132. In another example, access site information 138 may include a diagnosis or other classification of an access site, such as a healthy diagnosis, a grade or other classification level, indication of the presence and/or severity of an abnormality, and/or the like. For example, access site analysis application 150 may determine the presence and/or severity of an access site aneurysm using an access site analysis process according to some embodiments (for instance, using computational models 136).

In some embodiments, access site analysis application 150 may use one or more computational models 136 to analyze access site images 132 and/or access site description information 134 to determine access site information 138 and/or treatment recommendations 142. Non-limiting examples of computational models 136 may include an ML model, an AI model, a neural network (NN), an artificial neural network (ANN), a convolutional neural network (CNN), a deep learning (DL) network, a deep neural network (DNN), a recurrent neural network (RNNs), combinations thereof, variations thereof, and/or the like. Embodiments are not limited in this context. For example, a CNN may be used to analyze access site images 132 in which access site images 132 (or, more particularly, image files) are the input and access site information 138 (including, access site features, for example) and/or treatment recommendations may be the output.

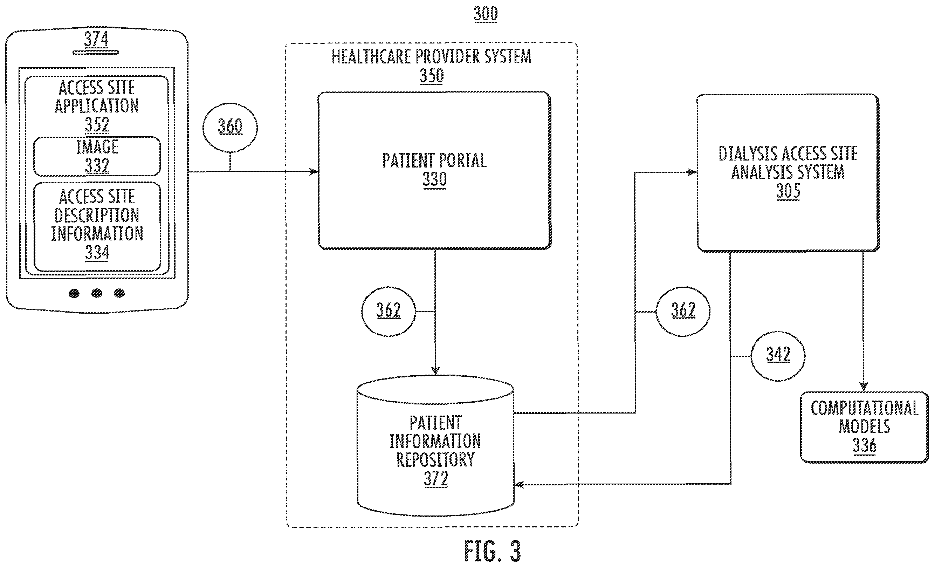

In various embodiments, access site analysis application 150 may use different computational models 136 for different portions of the access site analysis process. For example, an image-analysis computational model may be used to process access site images 132. In another example, a treatment recommendation computational model may be used to process access site information 138 and/or access site classification information 140 (see, for example, FIG. 3) to generate a treatment recommendation 142. In some embodiments, one computational model 136 may be used for analyzing access site images 132, access site description information 134, access site information 138, and/or access site classification information 140 to determine a treatment recommendation. Embodiments are not limited in this context.

Computational models 136 may include one or more models trained to analyze images and access site images 132 in particular. For example, in various embodiments, computational models 136 may be trained to analyze access site images 132 to determine access site features and/or other information that may be used to diagnose an access site using patient-based and/or population-based access site images. Computational models may include one or more models trained to analyze access site information 138 and/or access site classification information 140 to determine a treatment recommendation 142. For example, patient-based training may include training a computational model 136 with access site images 132 of a particular patient and information indicating the condition, abnormalities, or other information that may be used to determine access site information 138 and/or a treatment recommendation 142. In another example, population-based training may include training a computational model 136 with access site images 132 of a particular population of patients (for instance, geographic region, disease state, condition, different skin tones, different types of access sites, different ages of access sites, and/or the like) and information indicating the condition, abnormalities, or other information that may be used to determine access site information 138 and/or a treatment recommendation 142.

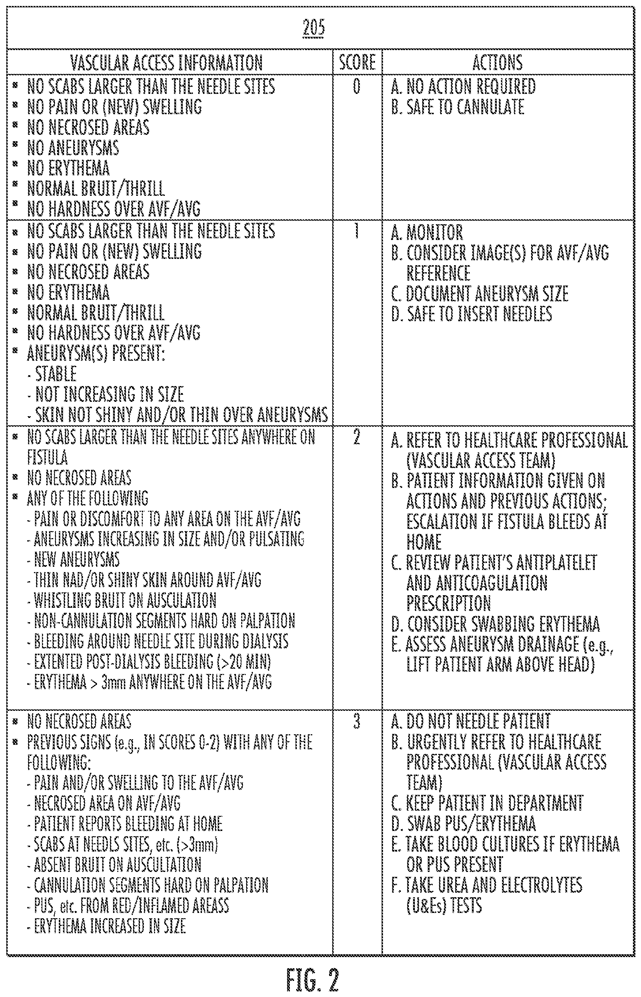

In various embodiments, access site classification information 140 may include information that may be used to classify, categorize, grade, or otherwise indicate the condition of an access site. **FIG. 2** depicts exemplary access site classification information according to some embodiments. As shown in FIG. 2, classification information 205 may include access site information (for instance, a Vascular Access Information), a Score, and Actions associated with each score classification. Vascular Access Information may include access site information 138 determined by access site analysis application 150 via analysis of access site images 132 and/or access site description information 134 using computational models 136. As depicted in FIG. 2, non-limiting examples of access site information 132 may include presence of scabs, scab properties, presence/severity of pain, presence/severity of swelling, new pain, new swelling, presence of necrosed areas, presence of erythema, bruit/thrill condition, AVF/AVG condition (for instance, hardness over AVF/AVG), presence of aneurysm, aneurysm characteristics (for instance, stable, increasing in size, skin condition over aneurysm, and/or the like), palpation information, and/or the like. Accordingly, some embodiments may operate to automatically classify the stages of an access site (for instance, AVF/AVG) aneurysm. Although a particular staging or Score and actions are depicted in FIG. 2, embodiments are not so limited, as the classification information 205 depicted in FIG. 2 is for illustrative purposes only. Other classifications, grading, scoring, and/or the like may be used according to some embodiments.

In various embodiments, access site analysis application 150 may analyze access site information 138 (for instance, information indicating the characteristics of the access site) based on access site information (for instance, Vascular Access Information) provided in access site classification information (for instance, classification information 205), to determine access site information 138 in the form of a diagnosis (for instance, a Score) and/or a treatment recommendation 142 (for instance, Actions). In some embodiments, access site analysis application 150 may determine access site information 138 (for instance, a diagnosis, score, and/or the like) and/or a treatment recommendation 142 (for instance, actions) using a computational model 136, a table lookup matching process, a pattern matching process, a search process, combinations thereof, and/or the like.

Access site analysis application 150 may generate treatment recommendations 142 based on access site information 138. Treatment recommendations 142 may include courses of action for treating and/or monitoring an access site. For example, a treatment recommendation 142 may indicate that an access site is safe for use (for instance, insertion of a needle) for a dialysis process. In another example, a treatment recommendation 142 may indicate instructions for clinical intervention, follow-up visits, limiting or eliminating use of access site, medication, additional actions to assess access site and/or abnormality condition, and/or the like. In various embodiments, treatment recommendations 142 may be provided to healthcare professionals for treatment of the patient, for example, via network 170 to a client device 174a-n and/or data store 172a-n accessible by a healthcare professional user. For example, the healthcare professional user may access a patient portal, an EMR system, or other interface to obtain patient information to review the access site images 132, access site description information 134, access site information, treatment recommendation 142, patient healthcare records including or relating to any of the foregoing, and/or the like.

**FIG. 3** illustrates an example of an operating environment 300 that may be representative of some embodiments. As shown in FIG. 3, operating environment 300 may include a patient computing device 374, such as a smartphone, a tablet computing device, a portable computing device, and/or the like. Computing device 374 may execute an access site application 352. In some embodiments, access site application 352 may be or may include a mobile application, client application, web-based application, and/or the like for interacting (for instance, directly or via a patient portal 330) with a dialysis access site analysis system 305 configured according to various embodiments.

A user may capture or otherwise access an image 332 of an access site or other portion of the patient. For example, a user may take one or more pictures and/or a video of the access site (for example, slowly moving the camera around the access site to obtain multiple views of the access site). In some embodiments, frames of a video of the access site may be converted into a plurality of images.

Access site application 352 may allow a user to enter access site description information 334 describing the image 332 and/or other personal characteristics. In some embodiments, access site application 352 may provide text boxes, check boxes (for example, to indicate the presence of a condition), selection objects, or other graphical user interface (GUI) objects for entering access site description information 334. In some embodiments, access site application 352 may facilitate the capture of image 332. For example, a user may open access site application 352 and access site application 352 may provide an image capture function (for instance, using a camera of computing device 374). In some embodiments, image 332 may include or may be associated with image information, including a size indicator, a color indicator, a shape selector, and/or the like. For example, a ruler or scale may be included in the image or may be used to determine size information. In another example, a color indicator may be used as a reference and/or to determine a color of a portion of the patient included in image 332. In a further example, a shape selector may be available to select, draw, or otherwise highlight a portion of the patient in image 332 (for example, a patient may draw a circle or other shape around the access site, source of pain, area of hardness, area of discoloration, area of change, and/or the like).

An image record 360 may be uploaded to a patient portal 330. In some embodiments, image record 360 may include image 332, access site description information 360, and/or other patient information. For example, image record 360 may include computing device 374 information, user identification information, user credential information, healthcare provider information, time stamp information, image quality information, and/or the like. In some embodiments, patient portal 330 may store image record 360 in a patient information repository 372. In some embodiments, repository 372 may be or may include a patient record database, such as a DIS, EMR system, and/or the like. In exemplary embodiments, patient portal 330 and patient information repository 372 may be part of a healthcare provider system 350. For example, patient portal 330 and patient information repository 372 may be used by a dialysis clinic or a plurality of dialysis clinics operated by a healthcare provider to provide patient care and manage patient healthcare information.

In various embodiments, patient portal 330 or other system may modify image record 360 to generate a modified image record 362. For example, for use as population-specific information, image record 362 may be de-identified of information that may be used to identify the patient associated with image record 360. In another example, the healthcare provider associated with patient portal 330 may include its own information, such as a data and time stamp of receipt of image record 360, changes made to image record 360, healthcare provider information, and/or the like. In some embodiments, image record 362 may be modified to be in a format corresponding to records and/or other information stored in repository 372.

Dialysis access site analysis system 305 may access image record 362 via healthcare provider system 350. For example, dialysis access site analysis system 305 may operate as a service to a healthcare provider, such as a subscription service and/or Software-as-a-Service (SaaS) provider. Dialysis access site analysis system 305 may analyze image record 362 according to some embodiments and generate a treatment recommendation 342 (see, for example, FIGS. 1, 4, and 6). In various embodiments, treatment recommendation 342 may be provided to the healthcare provider, for instance, by being stored in repository 372 with the patient records. In some embodiments, dialysis access site analysis system 305 may use image record 362 to train computational models 336. Alternatively, or in addition to, dialysis access site analysis system 305 may use other data to train computational models, such as the CMS database, USRDS database, third-party clinical data, in-silico clinical data, and/or the like.

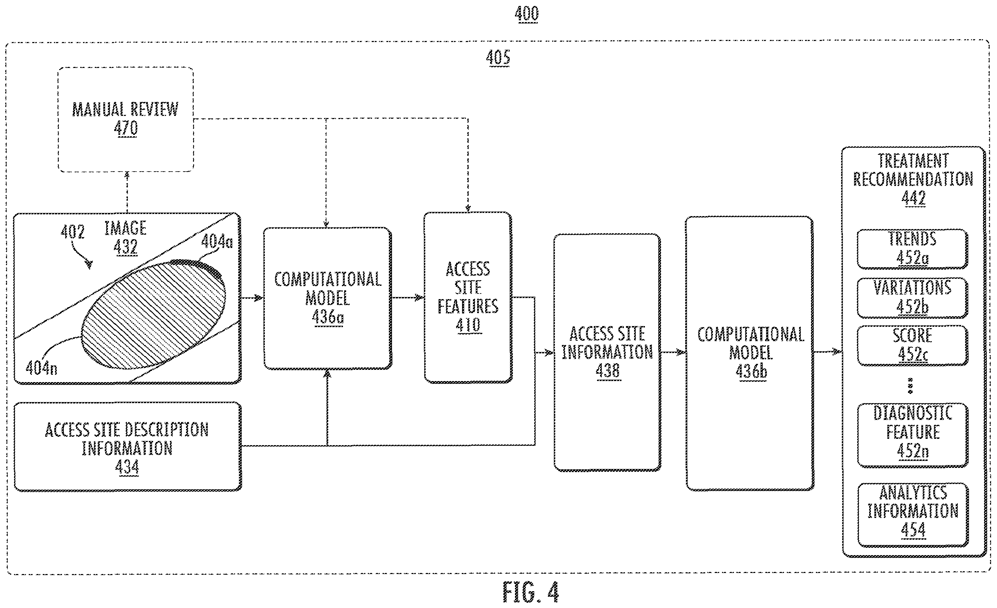

**FIG. 4** illustrates an example of an operating environment 400 that may be representative of some embodiments. As shown in FIG. 4, an access site analysis process 405 may include accessing an access site image 432 of an access site 402 having various elements 404a-n. For example, a first elements 404a may include scabbing and a second elements 404n may include a color of the access site. Access site description information 434 may also be accessed providing descriptive information associated with access site 402, such as symptoms, changes, vitals, and/or the like. Access site image 432 and/or access site description information 434 may be provided to a computational model 436a. In some embodiments, computational model 436a may include a CNN or other computational model operative to analyze access site images to determine access site features 410 based on analysis of the visual elements of access site image 432. For example, computational model 436a may be trained to determine a color or difference in color of areas of the access site (for instance, to look for redness, darkness, contrast with surrounding portions of the patient, and/or the like). In another example, computational model 436a may be trained to determine elements 404a-n within access site image 432, such as the access site, an aneurysm, areas of discoloration, areas of shiny skin, and/or the like. Embodiments are not limited in this context.

In various embodiments, computational model 436a may analyze access site image 432 alone or in combination with access site description information 434. For example, computational model 436a may detect a condition with a certain confidence level (for instance, inflammation). Computational model 436a may check access site description information 434 to determine whether inflammation has been indicated to increase the confidence level of the determination of inflammation as an access site feature and/or to train computational model 436a. In another example, computational model 436a may indicate areas of possible scabbing responsive to access site description information 434 describing scabbing in the access site.

In some embodiments, computational model 436a may compare access site image 432 to any previous images of the access site to determine certain access site features 410. In this manner, computational model 436a may determine trends (for instance, increasing element size, increasing inflammation, decreasing redness, decreasing shininess, and/or the like), variances (for example, presence new abnormality, absence of previous condition, color changes, shape changes, and/or the like), and other determinations that may be made based on viewing a series of images taking at different times.

In some embodiments, access site image 132 may undergo a manual review 470 by a healthcare professional. The results of manual review 470 may be provided to computational model 436a for analysis and/or training purposes and/or provided as access site features 410.

Access site features 410 and access site description information 434 may be provided as access site information 430 to computational model 436b. In various embodiments, computational model 436b may operate to determine a treatment recommendation 442 based on access site information 438. In some embodiments, computational model 436b may compare access site image 432 and/or access site information to any previous images or information associated with the access site to determine variations, trends, and/or the based on historical patient information.

In various embodiments, treatment recommendation 442 may include and/or may be based on one or more diagnostic features 452a-n including, without limitation, trends 452a, variations 452b, scores 452c, and/or the like. For example, a trend 452a may be determined that inflammation has been decreasing over the previous three image sample periods, indicating that treatment may be working. In another example, a variation 452b in color of the access site may indicate that a new condition or abnormality has developed. In a further example, a treatment recommendation 442 may include a score 452c or other categorization of the diagnosis (see, for example, FIG. 3).

In various embodiments, treatment recommendation 442 may include analytics information 454, for example, indicating outcomes, success rates, treatment types, and/or the like associated with other patients and/or populations of patients. For example, treatment recommendation 442 may include analytics information 454 indicating that Treatment A for Population B with Condition C had a success rate of 20% and Complications X, Y, and Z, while Treatment M for Population N with Condition C had a success rate of 30% with Complication X. A treatment recommendation 442 may be determined that is optimized for the patient as determined by computational model 436b. For example, computational model 436b may determine one or more most successful treatments (for instance, based on success rates) for patients with the same abnormality, in the same population group, in the same region, access site features, access site information, combinations thereof, and/or the like. Analytics information 454 may be provided based on relevance to the patient based on various patient characteristics, such as age, gender, access site type, access site age, abnormalities, diagnosis, and/or the like. For example, analytical information 454 may be provided that is relevant to the population group of the patient, type of access site, and/or the like. In this manner, a healthcare professional may more fully evaluate treatment recommendation 442 using population-based outcomes and success rates.

Included herein are one or more logic flows representative of exemplary methodologies for performing novel aspects of the disclosed architecture. While, for purposes of simplicity of explanation, the one or more methodologies shown herein are shown and described as a series of acts, those skilled in the art will understand and appreciate that the methodologies are not limited by the order of acts. Some acts may, in accordance therewith, occur in a different order and/or concurrently with other acts from that shown and described herein. For example, those skilled in the art will understand and appreciate that a methodology could alternatively be represented as a series of interrelated states or events, such as in a state diagram. Moreover, not all acts illustrated in a methodology may be required for a novel implementation. Blocks designated with dotted lines may be optional blocks of a logic flow.

A logic flow may be implemented in software, firmware, hardware, or any combination thereof. In software and firmware embodiments, a logic flow may be implemented by computer executable instructions stored on a non-transitory computer readable medium or machine readable medium. The embodiments are not limited in this context.

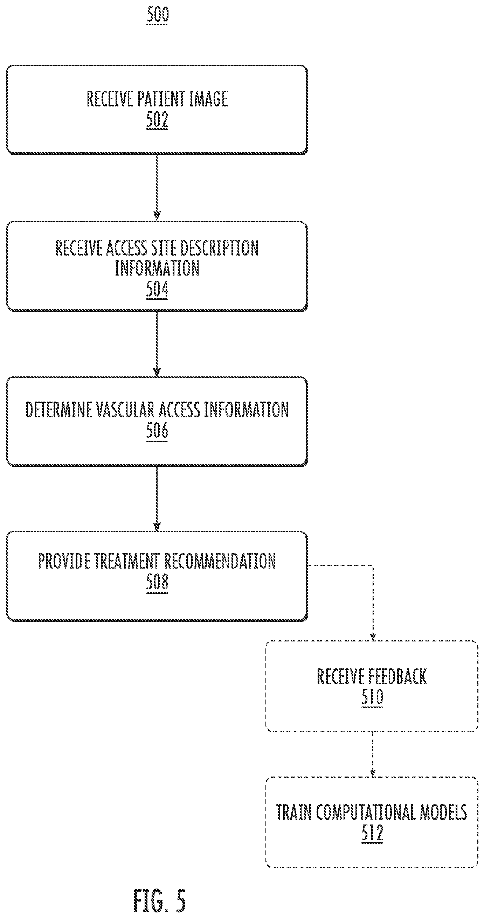

**FIG. 5** illustrates an embodiment of a logic flow 500. Logic flow 500 may be representative of some or all of the operations executed by one or more embodiments described herein, such as computing device 110. In some embodiments, logic flow 500 may be representative of some or all of the operations of an access site analysis process according to some embodiments.

At block 502, logic flow 500 may receive a patient image. For example, access site analysis application 150 may receive an access site image 132 stored in a data repository of a healthcare provider. Logic flow 500 may receive access site description information at block 504. For example, access site analysis application 150 may receive access site description information 134 associated with the patient image received in block 502. In some embodiments, the patient and the access site description information may be included in the same patient record stored, for example, in a healthcare provider database.

Logic flow 500 may determine access site information at block 506. For example, access site analysis application 150 may process an access site image 132 and/or access site description information 134 using a computational model configured according to some embodiments to determine vascular access information 138. In some embodiments, access site information 138 may include a diagnosis or other determination of the condition of the access site, including an indication of characteristics (color, size, abnormalities, and/or the like) and/or a categorization (for instance a score and associated actions). At block 508, logic flow 500 may provide a treatment recommendation. For example, access site analysis application 150 may generate a treatment recommendation 508 for the access site determined by processing the vascular access information using a computational model configured according to some embodiments. The treatment recommendation may include actions such as monitoring, healthcare provider evaluation, pharmaceuticals, continued/discontinued use of needles, and/or the like.

In some embodiments, logic flow 500 may receive feedback at block 510. For example, a healthcare provider may provide treatment outcomes and/or the like relating to a course of treatment for a patient and/or population of treatments, such as treatments associated with the treatment recommendation generated in block 508. In another example, a healthcare provider may provide feedback relating to the accuracy of the vascular access information, access site features, and/or the like generated by computational models 136. Feedback may be in various forms, such as images, textual description, clinical data, outcome information, and/or the like.

In various embodiments, logic flow 512 may train computational models at block 512. For example, access site analysis application 150 may train computational models 136 using the feedback received at block 510. In this manner, the computational models operative to determine access site information and/or treatment recommendations according to some embodiments may continually learn and improve their accuracy, confidence levels, breadth of analysis, and/or the like.

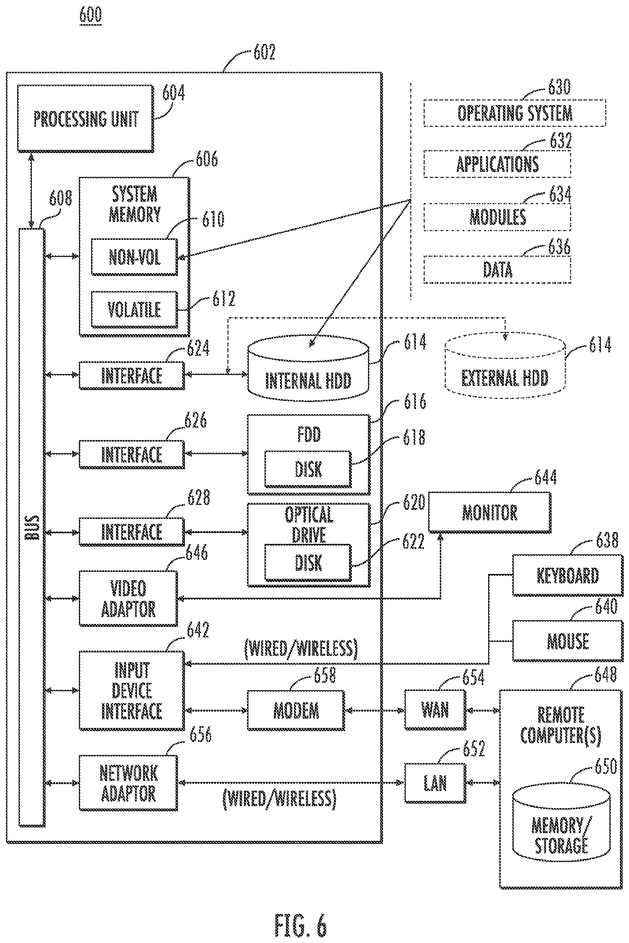

**FIG. 6** illustrates an embodiment of an exemplary computing architecture 600 suitable for implementing various embodiments as previously described. In various embodiments, the computing architecture 600 may comprise or be implemented as part of an electronic device. In some embodiments, the computing architecture 600 may be representative, for example, of computing device 110. The embodiments are not limited in this context.

As used in this application, the terms "system" and "component" and "module" are intended to refer to a computer-related entity, either hardware, a combination of hardware and software, software, or software in execution, examples of which are provided by the exemplary computing architecture 600. For example, a component can be, but is not limited to being, a process running on a processor, a processor, a hard disk drive, multiple storage drives (of optical and/or magnetic storage medium), an object, an executable, a thread of execution, a program, and/or a computer. By way of illustration, both an application running on a server and the server can be a component. One or more components can reside within a process and/or thread of execution, and a component can be localized on one computer and/or distributed between two or more computers. Further, components may be communicatively coupled to each other by various types of communications media to coordinate operations. The coordination may involve the uni-directional or bi-directional exchange of information. For instance, the components may communicate information in the form of signals communicated over the communications media. The information can be implemented as signals allocated to various signal lines. In such allocations, each message is a signal. Further embodiments, however, may alternatively employ data messages. Such data messages may be sent across various connections. Exemplary connections include parallel interfaces, serial interfaces, and bus interfaces.

The computing architecture 600 includes various common computing elements, such as one or more processors, multi-core processors, co-processors, memory units, chipsets, controllers, peripherals, interfaces, oscillators, timing devices, video cards, audio cards, multimedia input/output (I/O) components, power supplies, and so forth. The embodiments, however, are not limited to implementation by the computing architecture 600.

As shown in FIG. 6, the computing architecture 600 comprises a processing unit 604, a system memory 606 and a system bus 608. The processing unit 604 may be a commercially available processor and may include dual microprocessors, multi-core processors, and other multi-processor architectures.

The system bus 608 provides an interface for system components including, but not limited to, the system memory 606 to the processing unit 604. The system bus 608 can be any of several types of bus structure that may further interconnect to a memory bus (with or without a memory controller), a peripheral bus, and a local bus using any of a variety of commercially available bus architectures. Interface adapters may connect to the system bus 608 via a slot architecture. Example slot architectures may include without limitation Accelerated Graphics Port (AGP), Card Bus, (Extended) Industry Standard Architecture ((E)ISA), Micro Channel Architecture (MCA), NuBus, Peripheral Component Interconnect (Extended) (PCI(X)), PCI Express, Personal Computer Memory Card International Association (PCMCIA), and the like.

The system memory 606 may include various types of computer-readable storage media in the form of one or more higher speed memory units, such as read-only memory (ROM), random-access memory (RAM), dynamic RAM (DRAM), Double-Data-Rate DRAM (DDRAM), synchronous DRAM (SDRAM), static RAM (SRAM), programmable ROM (PROM), erasable programmable ROM (EPROM), electrically erasable programmable ROM (EEPROM), flash memory, polymer memory such as ferroelectric polymer memory, ovonic memory, phase change or ferroelectric memory, silicon-oxide-nitride-oxide-silicon (SONOS) memory, magnetic or optical cards, an array of devices such as Redundant Array of Independent Disks (RAID) drives, solid state memory devices (e.g., USB memory, solid state drives (SSD) and any other type of storage media suitable for storing information. In the illustrated embodiment shown in **FIG. 6****,** the system memory 606 can include non-volatile memory 610 and/or volatile memory 612. A basic input/output system (BIOS) can be stored in the non-volatile memory 610.

The computer 602 may include various types of computer-readable storage media in the form of one or more lower speed memory units, including an internal (or external) hard disk drive (HDD) 614, a magnetic floppy disk drive (FDD) 616 to read from or write to a removable magnetic disk 611, and an optical disk drive 620 to read from or write to a removable optical disk 622 (e.g., a CD-ROM or DVD). The HDD 614, FDD 616 and optical disk drive 620 can be connected to the system bus 608 by a HDD interface 624, an FDD interface 626 and an optical drive interface 628, respectively. The HDD interface 624 for external drive implementations can include at least one or both of Universal Serial Bus (USB) and IEEE 1114 interface technologies.

The drives and associated computer-readable media provide volatile and/or nonvolatile storage of data, data structures, computer-executable instructions, and so forth. For example, a number of program modules can be stored in the drives and memory units 610, 612, including an operating system 630, one or more application programs 632, other program modules 634, and program data 636. In one embodiment, the one or more application programs 632, other program modules 634, and program data 636 can include, for example, the various applications and/or components of computing device 110.

A user can enter commands and information into the computer 602 through one or more wired/wireless input devices, for example, a keyboard 638 and a pointing device, such as a mouse 640. These and other input devices are often connected to the processing unit 604 through an input device interface 642 that is coupled to the system bus 608, but can be connected by other interfaces.

A monitor 644 or other type of display device is also connected to the system bus 608 via an interface, such as a video adaptor 646. The monitor 644 may be internal or external to the computer 602. In addition to the monitor 644, a computer typically includes other peripheral output devices, such as speakers, printers, and so forth.

The computer 602 may operate in a networked environment using logical connections via wired and/or wireless communications to one or more remote computers, such as a remote computer 648. The remote computer 648 can be a workstation, a server computer, a router, a personal computer, portable computer, microprocessor-based entertainment appliance, a peer device or other common network node, and typically includes many or all of the elements described relative to the computer 602, although, for purposes of brevity, only a memory/storage device 650 is illustrated. The logical connections depicted include wired/wireless connectivity to a local area network (LAN) 652 and/or larger networks, for example, a wide area network (WAN) 654. Such LAN and WAN networking environments are commonplace in offices and companies, and facilitate enterprise-wide computer networks, such as intranets, all of which may connect to a global communications network, for example, the Internet.

The computer 602 is operable to communicate with wired and wireless devices or entities using the IEEE 802 family of standards, such as wireless devices operatively disposed in wireless communication (e.g., IEEE 802.16 over-the-air modulation techniques). This includes at least Wi-Fi (or Wireless Fidelity), WiMax, and Bluetooth^{™} wireless technologies, among others. Thus, the communication can be a predefined structure as with a conventional network or simply an ad hoc communication between at least two devices. Wi-Fi networks use radio technologies called IEEE 802.11x (a, b, g, n, etc.) to provide secure, reliable, fast wireless connectivity. A Wi-Fi network can be used to connect computers to each other, to the Internet, and to wire networks (which use IEEE 802.3-related media and functions).

Numerous specific details have been set forth herein to provide a thorough understanding of the embodiments. It will be understood by those skilled in the art, however, that the embodiments may be practiced without these specific details. In other instances, well-known operations, components, and circuits have not been described in detail so as not to obscure the embodiments. It can be appreciated that the specific structural and functional details disclosed herein may be representative and do not necessarily limit the scope of the embodiments.

Some embodiments may be described using the expression "coupled" and "connected" along with their derivatives. These terms are not intended as synonyms for each other. For example, some embodiments may be described using the terms "connected" and/or "coupled" to indicate that two or more elements are in direct physical or electrical contact with each other. The term "coupled," however, may also mean that two or more elements are not in direct contact with each other, but yet still co-operate or interact with each other.

Unless specifically stated otherwise, it may be appreciated that terms such as "processing," "computing," "calculating," "determining," or the like, refer to the action and/or processes of a computer or computing system, or similar electronic computing device, that manipulates and/or transforms data represented as physical quantities (e.g., electronic) within the computing system's registers and/or memories into other data similarly represented as physical quantities within the computing system's memories, registers or other such information storage, transmission or display devices. The embodiments are not limited in this context.

It should be noted that the methods described herein do not have to be executed in the order described, or in any particular order. Moreover, various activities described with respect to the methods identified herein can be executed in serial or parallel fashion.

Although specific embodiments have been illustrated and described herein, it should be appreciated that any arrangement calculated to achieve the same purpose may be substituted for the specific embodiments shown. This disclosure is intended to cover any and all adaptations or variations of various embodiments. It is to be understood that the above description has been made in an illustrative fashion, and not a restrictive one. Combinations of the above embodiments, and other embodiments not specifically described herein will be apparent to those of skill in the art upon reviewing the above description. Thus, the scope of various embodiments includes any other applications in which the above compositions, structures, and methods are used.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

As used herein, an element or operation recited in the singular and proceeded with the word "a" or "an" should be understood as not excluding plural elements or operations, unless such exclusion is explicitly recited. Furthermore, references to "one embodiment" of the present disclosure are not intended to be interpreted as excluding the existence of additional embodiments that also incorporate the recited features.

The present disclosure is not to be limited in scope by the specific embodiments described herein. Indeed, other various embodiments of and modifications to the present disclosure, in addition to those described herein, will be apparent to those of ordinary skill in the art from the foregoing description and accompanying drawings. Thus, such other embodiments and modifications are intended to fall within the scope of the present disclosure. Furthermore, although the present disclosure has been described herein in the context of a particular implementation in a particular environment for a particular purpose, those of ordinary skill in the art will recognize that its usefulness is not limited thereto and that the present disclosure may be beneficially implemented in any number of environments for any number of purposes. Accordingly, the invention is defined by the appended claims.

## Claims

1. An apparatus (100, 300, 400), comprising:
at least one processor (120, 604);
a memory (130, 606, 510, 612, 650) coupled to the at least one processor (120, 604), the memory (130, 606, 510, 612, 650) comprising instructions that, when executed by the at least one processor (120, 604), cause the at least one processor (120, 604) to:
receive an access site image (132, 332, 432) comprising at least one image of a dialysis access site (402, 404a-n, 410) of a patient,
determine access site information (138, 430) for the dialysis access site (402, 404a-n, 410) based on at least one access site feature (404a-n, 410) determined from the access site image (132, 332, 432), the access site information (138, 430) indicating a condition of the dialysis access site (402, 404a-n, 432),
determine a classification of the access site based on access site classification information (140, 205), and
determine a treatment recommendation (142, 342, 442, 508) for the dialysis access site (402, 404a-n, 410) based on the access site information (138, 430).

2. The apparatus (100, 300, 400) of claim 1, the instructions, when executed by the at least one processor (120, 604), to cause the at least one processor (120, 604) to:
receive access site description information (134, 334, 360, 434), and
determine the access site information (138, 430) based on the at least one access site feature (404a-n, 410) and the access site description information (134, 334, 360, 434).

3. The apparatus (100, 300, 400) of claim 1, the dialysis access site (402, 404a-n, 410) comprising one of an arteriovenous fistula (AVF) or an arteriovenous graft (AVG).

4. The apparatus (100, 300, 400) of claim 1, the instructions, when executed by the at least one processor (120, 604), to cause the at least one processor (120, 604) to provide the access site image (132, 332, 432) to a computational model (136, 336, 436a, 436b) to determine the at least one access site feature (404a-n, 410).

5. The apparatus (100, 300, 400) of claim 1, the at least one access site feature (404a-n, 410) comprising at least one of size, color, shape, or presence of an abnormality.

6. The apparatus (100, 300, 400) of claim 1, the access site image (132, 332, 432) captured via a client computing device.

7. The apparatus (100, 300, 400) of claim 1, the classification comprising a score and at least one treatment action.

8. The apparatus (100, 300, 400) of claim 1, the treatment recommendation (142, 342, 442, 508) comprising analytics information (454) indicating at least one treatment outcome associated with the treatment recommendation (142, 342, 442, 508).

9. A method implemented by computer executable instructions stored on a non-transistory computer readable medium and, when executed by a computing device (110), comprising:
receiving an access site image (132, 332, 432) comprising at least one image (132, 332, 432) of a dialysis access site (402, 404a-n, 410) of a patient;
determining access site information (138, 430) for the dialysis access site (402, 404a-n, 410) based on at least one access site feature (404a-n, 410) determined from the access site image (132, 332, 432), the access site information (138, 430) indicating a condition of the dialysis access site (402, 404a-n, 410);
determining a classification of the access site (402, 404a-n, 410) based on access site classification information (140, 205), and
determining a treatment recommendation (142, 342, 442, 508) for the dialysis access site (402, 404a-n, 410) based on the access site information (138, 430).

10. The method of claim 9, comprising:
receiving access site description information (134, 334, 360, 434); and
determining the access site information (138, 430) based on the at least one access site feature (404a-n, 410) and the access site description information (134, 334, 360, 434).

11. The method of claim 9, the dialysis access site (402, 404a-n, 410) comprising one of an arteriovenous fistula (AVF) or an arteriovenous graft (AVG).

12. The method of claim 9, comprising providing the access site image (132, 332, 432) to a computational model (136, 336, 436a, 436b) to determine the at least one access site feature (404a-n, 410).

13. The method of claim 9, the at least one access site feature (404a-n, 410) comprising at least one of size, color, shape, or presence of an abnormality.

14. The method of claim 9, the access site image (132, 332, 432) captured via a client computing device.

15. The method of claim 9, the classification comprising a score and at least one treatment action.

## Patentansprüche

1. Vorrichtung (100, 300, 400), umfassend:
mindestens einen Prozessor (120, 604);
einen an den mindestens einen Prozessor (120, 604) gekoppelten Speicher (130, 606, 510, 612, 650), wobei der Speicher (130, 606, 510, 612, 650) Anweisungen umfasst, welche, wenn sie von dem mindestens einen Prozessor (120, 604) ausgeführt werden, den mindestens einen Prozessor (120, 604) dazu veranlassen:
ein Zugangsstellenbild (132, 332, 432) zu empfangen, welches mindestens ein Bild von einer Dialyse-Zugangsstelle (402, 404a-n, 410) eines Patienten umfasst,
Zugangsstellen-Informationen (138, 430) für die Dialyse-Zugangsstelle (402, 404a-n, 410) basierend auf mindestens einem Zugangsstellen-Merkmal (404a-n, 410), welches ausgehend von dem Zugangsstellenbild (132, 332, 432) ermittelt wurde, zu ermitteln, wobei die Zugangsstellen-Informationen (138, 430) einen Zustand der Dialyse-Zugangsstelle (402, 404a-n, 432) anzeigen,
eine Klassifizierung der Zugangsstelle basierend auf Zugangsstellen-Klassifizierungs-Informationen (140, 205) zu ermitteln und
eine Behandlungsempfehlung (142, 342, 442, 508) für die Dialyse-Zugangsstelle (402, 404a-n, 410) basierend auf den Zugangsstellen-Informationen (138, 430) zu ermitteln.

2. Vorrichtung (100, 300, 400) nach Anspruch 1, wobei die Anweisungen, wenn sie von dem mindestens einen Prozessor (120, 604) ausgeführt werden, den mindestens einen Prozessor (120, 604) dazu veranlassen:
Zugangsstellen-Beschreibungs-Informationen (134, 334, 360, 434) zu empfangen und
die Zugangsstellen-Informationen (138, 430) basierend auf dem mindestens einen Zugangsstellen-Merkmal (404a-n, 410) und den Zugangsstellen-Beschreibungs-Informationen (134, 334, 360, 434) zu ermitteln.

3. Vorrichtung (100, 300, 400) nach Anspruch 1, wobei die Dialyse-Zugangsstelle (402, 404a-n, 410) eine arteriovenöse Fistel (AVF) oder ein arteriovenöses Transplantat (AVG) umfasst.

4. Vorrichtung (100, 300, 400) nach Anspruch 1, wobei die Anweisungen, wenn sie von dem mindestens einen Prozessor (120, 604) ausgeführt werden, den mindestens einen Prozessor (120, 604) dazu veranlassen, das Zugangsstellenbild (132, 332, 432) einem Rechenmodell (136, 336, 436a, 436b) bereitzustellen, um das mindestens eine Zugangsstellen-Merkmal (404a-n, 410) zu ermitteln.

5. Vorrichtung (100, 300, 400) nach Anspruch 1, wobei das mindestens eine Zugangsstellen-Merkmal (404a-n, 410) mindestens eines der folgenden umfasst: Größe, Farbe, Form oder Vorhandensein einer Abnormalität.

6. Vorrichtung (100, 300, 400) nach Anspruch 1, wobei das Zugangsstellenbild (132, 332, 432) über ein Client-Computergerät erfasst wird.

7. Vorrichtung (100, 300, 400) nach Anspruch 1, wobei die Klassifizierung ein Ergebnis und mindestens eine Behandlungsmaßnahme umfasst.

8. Vorrichtung (100, 300, 400) nach Anspruch 1, wobei die Behandlungsempfehlung (142, 342, 442, 508) Analytikinformationen (454) umfasst, welche mindestens ein mit der Behandlungsempfehlung (142, 342, 442, 508) verbundenes Behandlungsergebnis anzeigen.

9. Verfahren, welches durch computerausführbare, auf einem nicht flüchtigen computerlesbaren Medium gespeicherte Anweisungen implementiert wird und, wenn es von einem Computergerät (110) ausgeführt wird, Folgendes umfasst:
Empfangen eines Zugangsstellenbilds (132, 332, 432), welches mindestens ein Bild (132, 332, 432) einer Dialyse-Zugangsstelle (402, 404a-n, 410) eines Patienten umfasst;
Ermitteln von Zugangsstellen-Informationen (138, 430) für die Dialyse-Zugangsstelle (402, 404a-n, 410) basierend auf mindestens einem Zugangsstellen-Merkmal (404a-n, 410), welches ausgehend von dem Zugangsstellenbild (132, 332, 432) ermittel wurde, wobei die Zugangsstellen-Informationen (138, 430) einen Zustand der Dialyse-Zugangsstelle (402, 404a-n, 410) anzeigen;
Ermitteln einer Klassifizierung der Zugangsstelle (402, 404a-n, 410) basierend auf Zugangsstellen-Klassifizierungs-Informationen (140, 205) und
Ermitteln einer Behandlungsempfehlung (142, 342, 442, 508) für die Dialyse-Zugangsstelle (402, 404a-n, 410) basierend auf den Zugangsstellen-Informationen (138, 430).

10. Verfahren nach Anspruch 9, umfassend:
Empfangen von Zugangsstellen-Beschreibungs-Informationen (134, 334, 360, 434) und
Ermitteln der Zugangsstellen-Informationen (138, 430) basierend auf dem mindestens einen Zugangsstellen-Merkmal (404a-n, 410) und den Zugangsstellen-Beschreibungs-Informationen (134, 334, 360, 434).

11. Verfahren nach Anspruch 9, wobei die Dialyse-Zugangsstelle (402, 404a-n, 410) eine arteriovenöse Fistel (AVF) oder ein arteriovenöses Transplantat (AVG) umfasst.

12. Verfahren nach Anspruch 9, umfassend Bereitstellen des Zugangsstellenbildes (132, 332, 432) an ein Rechenmodell (136, 336, 436a, 436b), um das mindestens eine Zugangsstellen-Merkmal (404a-n, 410) zu ermitteln.

13. Verfahren nach Anspruch 9, wobei das mindestens eine Zugangsstellen-Merkmal (404a-n, 410) mindestens eines der folgenden umfasst: Größe, Farbe, Form oder Vorhandensein einer Abnormalität.

14. Verfahren nach Anspruch 9, wobei das Zugangsstellenbild (132, 332, 432) über ein Client-Computergerät erfasst wird.

15. Verfahren nach Anspruch 9, wobei die Klassifizierung ein Ergebnis und mindestens eine Behandlungsmaßnahme umfasst.

## Revendications

1. Appareil (100, 300, 400), comprenant :
au moins un processeur (120, 604) ;
une mémoire (130, 606, 510, 612, 650) couplée à l'au moins un processeur (120, 604), la mémoire (130, 606, 510, 612, 650) comprenant des instructions qui, lorsqu'elles sont exécutées par l'au moins un processeur (120, 604), amènent l'au moins un processeur (120, 604) à :
recevoir une image de site d'accès (132, 332, 432) comprenant au moins une image d'un site d'accès de dialyse (402, 404a-n, 410) d'un patient,
déterminer des informations de site d'accès (138, 430) pour le site d'accès de dialyse (402, 404a-n, 410) sur la base d'au moins une caractéristique de site d'accès (404a-n, 410) déterminée à partir de l'image du site d'accès (132, 332, 432), les informations de site d'accès (138, 430) indiquant un état du site d'accès de dialyse (402, 404a-n, 432),
déterminer une classification du site d'accès sur la base des informations de classification du site d'accès (140, 205), et
déterminer une recommandation de traitement (142, 342, 442, 508) pour le site d'accès de dialyse (402, 404a-n, 410) sur la base des informations du site d'accès (138, 430).

2. Appareil (100, 300, 400) selon la revendication 1, dans lequel les instructions, lorsqu'elles sont exécutées par l'au moins un processeur (120, 604), amènent l'au moins un processeur (120, 604) à :
recevoir des informations de description du site d'accès (134, 334, 360, 434), et
déterminer les informations du site d'accès (138, 430) sur la base de l'au moins une caractéristique du site d'accès (404a-n, 410) et des informations de description du site d'accès (134, 334, 360, 434).

3. Appareil (100, 300, 400) selon la revendication 1, le site d'accès de dialyse (402, 404a-n, 410) comprenant l'une d'une fistule artério-veineuse (AVF) ou d'un greffon artério-veineux (AVG).

4. Appareil (100, 300, 400) selon la revendication 1, dans lequel les instructions, lorsqu'elles sont exécutées par l'au moins un processeur (120, 604), amènent l'au moins un processeur (120, 604) à fournir l'image du site d'accès (132, 332, 432) à un modèle informatique (136, 336, 436a, 436b) pour déterminer l'au moins une caractéristique du site d'accès (404a-n, 410).

5. Appareil (100, 300, 400) selon la revendication 1, dans lequel l'au moins une caractéristique du site d'accès (404a-n, 410) comprend au moins un élément parmi la taille, la couleur, la forme ou la présence d'une anomalie.

6. Appareil (100, 300, 400) selon la revendication 1, dans lequel l'image du site d'accès (132, 332, 432) est capturée par l'intermédiaire d'un dispositif informatique client.

7. Appareil (100, 300, 400) selon la revendication 1, dans lequel la classification comprend un score et au moins une action de traitement.

8. Appareil (100, 300, 400) selon la revendication 1, dans lequel la recommandation de traitement (142, 342, 442, 508) comprend des informations analytiques (454) indiquant au moins un résultat de traitement associé à la recommandation de traitement (142, 342, 442, 508).

9. Procédé mis en œuvre par des instructions exécutables par ordinateur stockées sur un support non-transitoire lisible par ordinateur, le procédé, lorsqu'il est exécuté par un dispositif informatique (110), comprenant les étapes suivantes :
recevoir une image de site d'accès (132, 332, 432) comprenant au moins une image (132, 332, 432) d'un site d'accès de dialyse (402, 404a-n, 410) d'un patient ;
déterminer des informations de site d'accès (138, 430) pour le site d'accès de dialyse (402, 404a-n, 410) sur la base d'au moins une caractéristique de site d'accès (404a-n, 410) déterminée à partir de l'image du site d'accès (132, 332, 432), les informations de site d'accès (138, 430) indiquant un état du site d'accès de dialyse (402, 404a-n, 410) ;
déterminer une classification du site d'accès (402, 404a-n, 410) sur la base des informations de classification du site d'accès (140, 205), et
déterminer une recommandation de traitement (142, 342, 442, 508) pour le site d'accès de dialyse (402, 404a-n, 410) sur la base des informations sur le site d'accès (138, 430).

10. Procédé selon la revendication 9, comprenant en outre les étapes suivantes :
recevoir des informations de description du site d'accès (134, 334, 360, 434), et
déterminer les informations du site d'accès (138, 430) sur la base de l'au moins une caractéristique du site d'accès (404a-n, 410) et des informations de description du site d'accès (134, 334, 360, 434).

11. Procédé selon la revendication 9, le site d'accès de dialyse (402, 404a-n, 410) comprenant l'une d'une fistule artério-veineuse (AVF) ou d'un greffon artério-veineux (AVG).

12. Procédé selon la revendication 9, comprenant de fournir l'image du site d'accès (132, 332, 432) à un modèle informatique (136, 336, 436a, 436b) pour déterminer l'au moins une caractéristique du site d'accès (404a-n, 410).

13. Procédé selon la revendication 9, dans lequel l'au moins une caractéristique du site d'accès (404a-n, 410) comprend au moins un élément parmi la taille, la couleur, la forme ou la présence d'une anomalie.

14. Procédé selon la revendication 9, dans lequel l'image du site d'accès (132, 332, 432) est capturée par l'intermédiaire d'un dispositif informatique client.

15. Procédé selon la revendication 9, dans lequel la classification comprend un score et au moins une action de traitement.
